(19) **Europäisches Patentamt**

**European Patent Office**

**Office européen des brevets**

(11) **EP 1 327 457 B1**

(12) **EUROPÄISCHE PATENTSCHRIFT**

(45) Veröffentlichungstag und Bekanntmachung des Hinweises auf die Patenterteilung:
**25.01.2006 Patentblatt 2006/04**

(51) Int Cl.:
*A61M 1/16* *(2006.01)*

(21) Anmeldenummer: **02026046.9**

(22) Anmeldetag: **22.11.2002**

(54) **Vorrichtung zur Detektion einer Leckage in einem Flüssigkeitssystem einer Blutbehandlungsvorrichtung**

Device to detect a leak in a liquid circuit of a blood treatment apparatus

Dispositif pour détecter une fuite dans un circuit de liquide d'un appareil de traitement du sang

(84) Benannte Vertragsstaaten:
**AT BE BG CH CY CZ DE DK EE ES FI FR GB GR IE IT LI LU MC NL PT SE SK TR**

(30) Priorität: **15.01.2002 DE 10201109**

(43) Veröffentlichungstag der Anmeldung:
**16.07.2003 Patentblatt 2003/29**

(73) Patentinhaber: **Fresenius Medical Care Deutschland GmbH 61352 Bad Homburg v.d.H. (DE)**

(72) Erfinder:
• **Balschat, Klaus 97525 Schwebheim (DE)**
• **Ender, Helmuth 97475 Zeil am Main (DE)**
• **Gagel, Alfred 96123 Litzendorf (DE)**
• **Spickermann, Reiner 97535 Wasserlose- Burghausen (DE)**

(74) Vertreter: **Luderschmidt, Schüler & Partner GbR Patentanwälte, John-F.-Kennedy-Strasse 4 65189 Wiesbaden (DE)**

(56) Entgegenhaltungen:
**DE-A- 4 239 937     US-A- 5 431 811**

**Beschreibung**

Die Erfindung betrifft eine Vorrichtung zur Detektion einer Leckage in einem Flüssigkeitssystem einer Blutbehandlungsvorrichtung mit einem extrakorporalen Blutkreislauf, insbesondere einer Blutbehandlungsvorrichtung, die einen von einer semipermeablen Membran in eine Blutkammer und eine Dialysierflüssigkeitskammer unterteilten Dialysator aufweist.

Hämodialysevorrichtungen mit einer Ultrafiltration(UF)-Kontrolleinrichtung haben weite Verbreitung gefunden. Die UF-Kontrolleinrichtung erlaubt es, eine bestimmte Ultrafiltrationsmenge vorzugeben. Es ist bekannt, daß eine UF-Kontrolleinrichtung ihr Vermögen, die Ultrafiltrationsrate exakt zu steuern, durch einen Defekt verlieren kann. Da eine durch einen solchen Defekt drastisch erhöhte oder verminderte Ultrafiltrationsrate zu einer Gefährdung des Patienten führen kann, verlangen bekannte Sicherheitsnormen, daß ein Schutzsystem vorhanden ist, das eine für den Patienten gefährliche Ultrafiltration verhindert. Als ein solches Schutzsystem wird eine Überwachung des Transmembrandrucks (TMP) akzeptiert.

In der Praxis darf die Fehlbilanzierung bei der Ultrafiltration maximal 500 ml während einer Behandlung betragen. Bei einer Behandlungsdauer von 4 h entspricht dies einem mittleren Volumenstrom von nur 125 ml/h.

Die Entwicklung von Dialysatoren mit Membranen hoher Permeabilität, sog. High-Flux-Dialysatoren, hat jedoch dazu geführt, daß eine Überwachung des TMP ein gefährlich hohe oder niedrige Ultrafiltrationsrate nicht mit hinreichender Auflösung erkennen kann, bedingt durch die beschränkte Auflösung des TMP-Sensors.

Um wenigstens sicher zu stellen, daß die Behandlung mit einem intakten UF-Kontrollsystem beginnt, sind Einrichtungen auf dem Markt, die eine manuelle oder selbständige Prüfung der Integrität des Kontrollsystems vor der Behandlung erlauben. Diese Prüfung erfolgt mit einem Druckhaltetest im Dialyseteil des Geräts.

Die DE 42 39 937 A1 beschreibt ein Verfahren und eine Vorrichtung zur Detektion einer Leckage in dem Flüssigkeitssystem einer Hämodialysevorrichtung auf der Grundlage eines Druckhaltetests, der die Feststellung der Funktionsfähigkeit des Kontrollsystems für die Ultrafiltrationsrate auch während der Dialysebehandlung ermöglicht. Zur Durchführung des Druckhaltetests wird während der Dialyse in periodischen Zeitabständen der Dialysator für jeweils ein kurzes Zeitintervall vom Dialysierflüssigkeitsteil der Hämodialysevorrichtung abgetrennt, und der Druckverlauf in der Dialysierflüssigkeit außerhalb des abgetrennten Dialysators wird im Sinne eines Druckhaltetests auf Abweichung vom stabilen Zustand erfaßt. Dieses Verfahren hat sich in der Praxis bewährt, als nachteilig erweist sich jedoch, daß für den Druckhaltetest die Dialysebehandlung in regelmäßigen Abständen unterbrochen werden muß. Darüber hinaus ist nachteilig, daß ein Leck quantitativ nicht erfaßt werden kann.

Die US 5,431,811 A beschreibt eine Dialysevorrichtung, bei der im Dialysierflüssigkeitskreislauf ein Filter zum Filtern der dem Dialysator zufließenden Dialysierflüssigkeit angeordnet ist. Stromauf und stromab des Filters sind Drucksensoren zur Messung des Transmembrandrucks vorgesehen. Für den Fall, dass der Transmembrandruck einen vorgegebenen Grenzwert überschreitet, wird ein Alarm gegeben. Die bekannte Dialysevorrichtung erlaubt nicht die Erkennung von Leckagen im Flüssigkeitssystem.

Der Erfindung liegt die Aufgabe zugrunde, eine Vorrichtung anzugeben, die eine Detektion von Leckagen im Flüssigkeitssystem einer Blutbehandlungsvorrichtung grundsätzlich ohne Unterbrechung der Blutbehandlung erlaubt.

Die Lösung dieser Aufgabe erfolgt erfindungsgemäß mit den Merkmalen des Patentanspruchs 1.

Die erfindungsgemäße Vorrichtung zur Detektion einer Leckage beruht auf einer fortlaufenden Messung des Drucks im Flüssigkeitssystem der Blutbehandlungsvorrichtung während eines vorgegebenen Zeitraums der Behandlungsdauer, wobei dieser Zeitraum einen Teil der Behandlungsdauer oder auch die gesamte Dauer der Behandlung ausmachen kann.

Es hat sich gezeigt, daß vor allem die Änderung des statischen Drucks im Flüssigkeitssystem der Blutbehandlungsvomchtung Aufschluß über das Vorliegen einer Leckage gibt. Der Druck kann prinzipiell an jeder Stelle des Flüssigkeitssystems gemessen werden. Bei einer Hämodialysevorrichtung mit einem Dialysator, der von einer semipermeablen Membran in eine Blutkammer und eine Dialysierflüssigkeitskammer unterteilt ist, sind Messungen des Drucks in der Dialysierflüssigkeitsleitung stromauf und/oder stromab der Dialysierflüssigkeitskammer des Dialysators möglich. Auch sind Messungen stromauf und/oder stromab der Blutkammer in der Blutleitung möglich. Allein entscheidend ist, daß die Änderung des Drucks während der Blutbehandlung kontinuierlich erfaßt wird.

Aus der Änderung des Drucks werden während der Blutbehandlung die Leckraten in vorgegebenen Zeitintervallen des vorgegebenen Zeitraums der Behandlungsdauer bestimmt. Aus den Leckraten wird dann das Leckage-Volumen in dem vorgegebenen Zeitraum der Behandlungsdauer ermittelt. Die Bestimmung des Leckage-Volumens erfolgt also durch Integration der kontinuierlich ermittelten Leckraten. Damit steht die gesamte Leckrnenge jederzeit quantitativ zur Verfügung. Das Leckage-Volumen wird daraufhin mit einem Grenzwert verglichen. Bei Überschreiten des Grenzwertes wird auf einen möglichen Störfall geschlossen.

Zur Bestimmung der Änderung des Drucks während des vorgegebenen Zeitraums wird vorzugsweise fortlaufend jeweils ein Druckwert in aufeinander folgenden Takten bestimmt und mit einem Bezugswert des Druckes verglichen. Bei einem plötzlich auftretenden Leck ändern sich die Drucksignale sehr schnell in einem typischen Bereich von einigen Sekunden bis zu einer Minute. Leckagen werden dann sicher erkannt, wenn die Verzögerungszeit Δt zwischen dem Druckwert und dem Bezugswert des Drucks ebenfalls einige Minuten beträgt. Als Bezugswert des Druckes sollte der jeweils in einem vorherigen Takt gemessene Druckwert sein.

Damit können langsame Druckänderungen aufgrund von Parameterdrifts eliminiert werden. Bei schleichenden Lecks hingegen ist es möglich, daß ein zunächst dichtes System eine kleine Leckrate aufweist, die im Laufe der Zeit stetig ansteigt. Entscheidend dabei ist nicht der Gradient, sondern die Dauer der Änderung. Schleichende Lecks können dann sicher erkannt werden, wenn der Bezugswert des Druckes der Druck zu Beginn des vorgegebenen Zeitraums ist, d. h. der Bezugswert des Druckes konstant ist.

Der zu bestimmende Druckwert kann der momentan gemessene Druck im Flüssigkeitssystem sein. Vorteilhaft ist, wenn zur Bestimmung des Druckwertes eine Vielzahl von Messungen in einem vorgegebenen Zeitintervall statistisch ausgewertet werden. Vorzugsweise ist der Druckwert ein Mittelwert, der in einem Mittelungsintervall gemessenen Druckwerte. Das Mittlungsintervall sollte dabei eine ausreichende Länge haben, um charakteristische Meßgrößen zu erzielen.

Das gemessene Drucksignal ist im allgemeinen von verschiedenen Störsignalen überlagert, die insbesondere auf den Betrieb der Dialysierflüssigkeits- und Blutpumpe, den Betrieb der Ultrafiltrationspumpe sowie der Umschaltung der Bilanzkammem zurückzuführen sind. Zur Elimination dieser Störgrößen können die verschiedensten Verfahren angewandt werden. Da die Störgrößen einen charakteristischen Signalverlauf haben, können diese erkannt und ausgeblendet werden. Entscheidend ist, daß zur Bestimmung der Druckänderung ein Drucksignal herangezogen wird, daß möglichst frei von Störgrößen ist.

Wenn das Leckage-Volumen den vorgegebenen Grenzwert überschreitet, kann ein Alarm gegeben werden, der auf einen möglichen Störfall hinweist. Es ist aber auch möglich, daß ein Störfall erst dann angenommen wird, wenn auf der Grundlage des bekannten Druckhaltetests Undichtigkeiten im Flüssigkeitssystem festgestellt werden. Hierzu wird bei Überschreiten des vorgegebenen Grenzwertes die Blutbehandlung unterbrochen und der Druckhaltetest mit entsprechender Empfindlichkeit durchgeführt. Damit findet eine Unterbrechung der Blutbehandlung erst dann statt, wenn eine Leckage mit hoher Wahrscheinlichkeit vorliegt. Bei der Feststellung von Undichtigkeiten mittels des Druckhaltetests kann ein akustischer und/oder optischer Alarm gegeben werden. Es kann auch ein Eingriff in die Blutbehandlung vorgenommen werden, wobei die Blutbehandlung vorzugsweise unterbrochen wird.

Sollten bei dem Druckhaltetest jedoch Undichtigkeiten nicht festgestellt werden können, wird die Überwachung des Flüssigkeitssystem auf der Grundlage der fortlaufenden Druckmessung vorzugsweise wieder eingeleitet.

Der Druckwert kann aus dem an einer oder mehreren Stellen des Flüssigkeitssystems gemessenen Druckes bestimmt werden. Einen besonders großen Einfluß haben Leckagen auf den Mittelwert zwischen dem Druck in der Dialysierflüssigkeit stromauf und stromab des Dialysators. Darüber hinaus kann auch der Transmembrandruck herangezogen werden. Auch kann der Druck nur auf der Blutseite gemessen werden. So ist beispielsweise die Erkennung von Leckagen nur mit einer Messung des Drucks im Blut stromab der Blutkammer möglich, der vom Druckabfall beispielsweise an der Nadel aufgrund des zum Patienten zurückströmenden Bluts beeinflußt wird. Bei einem Leck fließt ein Teil des flüssigen Blutstroms über die Membran auf die Dialysatseite, wodurch der Blutfluß um diesen Betrag verringert wird, so daß der Druck im Blutstrom stromab der Blutkammer fällt.

Prinzipiell ist es auch möglich, der Überwachung mehrere auf unterschiedlichen Druckmessungen basierende Druckwerte zu Grunde zu legen. Damit ist eine Plausibilitätsüberprüfung möglich. Beispielsweise kann auf eine Leckage insbesondere dann geschlossen werden, wenn der Transmembrandruck ansteigt und der Druck im Blutstrom stromab der Blutkammer fällt.

Es hat sich gezeigt, daß die Ultrafiltrationsrate und die Leckrate gleichwertige Störungen für den statischen Druck sind. Daher kann der Einfluß einer Leckage auf den Druck im Flilssigkeitssystem nach Betrag und Richtung durch Variation der UF-Rate vor der Behandlung simuliert werden. Während der Behandlung können die Meßwerte dann entsprechend verglichen werden.

Die Vorrichtung zur Detektion einer Leckage verfügt über eine Steuer- und Recheneinheit, Mittel zum Erfassen der Druckmesswerte, Mittel zum Bestimmen der Leckraten und Mittel zum Bestimmen des Leckage-Volumens sowie Mittel zum Vergleichen des Leckage-Volumens mit einem Grenzwert. Derartige Mittel können durch einen Mikroprozessor mit entsprechender Software sowie geeignete Drucksensoren bereitgestellt werden. Da ein Mikroprozessor und Drucksensoren in den bekannten Blutbehandlungsvorrichtungen vorhanden sind, ist der apparative Aufwand gering.

Im folgenden wird ein Ausführungsbeispiel der Erfindung unter Bezugnahme auf die Zeichnungen näher erläutert.

Es zeigen:

Figur 1

Die wesentlichen Komponenten einer Hämodialysevorrichtung mit einer Vorrichtung zur Detektion einer Leckage in schematische a Darstellung,

Figur 2

den zeitlichen Verlauf des ermittelten Druckwertes,

Figur 3
den zeitlichen Verlauf des ermittelten Mittelwerts des Druckwertes,

Figur 4
den zeitlichen Druckverlauf von PD1 und PD2,

Figur 5
den zeitlichen Verlauf des Transmembrandrucks und des korrigierten Transmembrandrucks TMP2,

Figur 6
den zeitlichen Verlauf des Drucks PDm und des korrigierten Drucks PDm und

Figur 7
den zeitlichen Verlauf des Drucks PB2 und des korrigierten Drucks PB2.

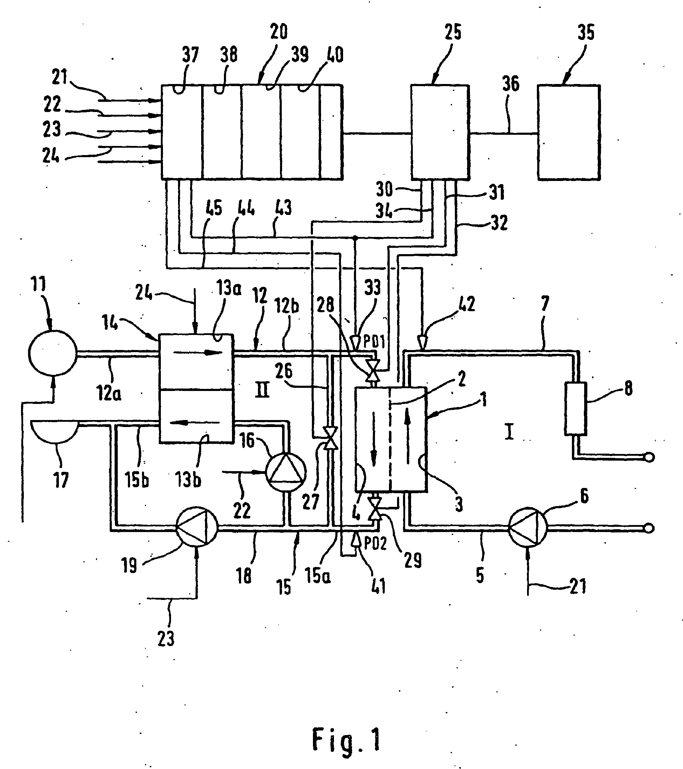

Figur 1 zeigt in schematischer Darstellung die wesentlichen Komponenten einer Hämodialysevorrichtung zusammen mit einer Vorrichtung zur Detektion einer Leckage in dem Flüssigkeitssystem der Dialysevorrichtung.

Die Hämodialysevorrichtung umfaßt einen extrakorporalen Blutkreislauf I und ein Dialysierflüssigkeitssystem II. Der extrakorporale Blutkreislauf I und das Dialysierflüssigkeitssystem II bilden das Flüssigkeitssystem der Dialysevorrichtung.

Die Dialysevorrichtung weist einen Dialysator 1 auf, der durch eine semipermeable Membran 2 in eine Blutkammer 3 und eine Dialysierflüssigkeitskammer 4 getrennt ist. Der Einlaß der Blutkammer ist mit einem Ende einer Blutzuführleitung 5 verbunden, in die eine Blutpumpe 6 geschaltet ist, während der Auslaß der Blutkammer 3 mit einem Ende einer Blutabführleitung 7 verbunden ist, in die eine Tropfkammer 8 geschaltet ist. Dieser Teil des Flüssigkeitssystems stellt den extrakorporalen Blutkreislauf I der Dialysevorrichtung dar.

Das Dialysierflüssigkeitssystem II der Dialysevorrichtung umfaßt eine Einrichtung 11 zur Bereitstellung von Dialysierflüssigkeit. Sie ist über einen ersten Abschnitt 12a einer Dialysierflüssigkeitzuführleitung 12 mit dem Einlaß der ersten Kammerhälfte 13a einer Bilanziereinrichtung 14 verbunden. Der zweite Abschnitt 12b der Dialysierflüssigkeitzuführleitung 12 verbindet den Auslaß der ersten Bilanzierkammerhälfte 13a mit dem Einlaß der Dialysierflüssigkeitskammer 4. Der Auslaß der Dialysierflüssigkeitskammer 4 ist über den ersten Abschnitt 15a einer Dialysierflüssigkeitsabführleitung 15 mit dem Einlaß der zweiten Bilanzierkammerhälfte 13b der Bilanziereinrichtung 14 verbunden. In den ersten Abschnitt 15a der Dialysierflüssigkeitabführleitung 15 ist eine Dialysierflüssigkeitspumpe 16 geschaltet. Der Auslaß der zweiten Bilanzierkammerhälfte 13b ist über den zweiten Abschnitt 15b der Dialysierflüssigkeitsabführleitung 15 mit einem Auslauf 17 verbunden. Stromauf der Dialysierflüssigkeitspumpe 16 zweigt von der Dialysierflüssigkeitsabführleitung 15 eine Ultrafiltratleitung 18 ab, die ebenfalls zu dem Auslauf 17 führt. In die Ultranitratleituhg 18 ist eine Ultrafiltrationspumpe 19 geschaltet.

In Figur 1 sind der besseren Übersichtlichkeit halber nur die beiden Kammerhälften der ersten Bilanzkammer dargestellt. Die Bilanziereinrichtung 14 weist aber noch eine zweite Bilanzkammer auf, die der ersten Kammer parallel geschaltet ist. Darüber hinaus weist die Bilanziereinrichtung ebenfalls nicht dargestellte Ventile zur Steuerung des Dialysierflüssigkeitsflusses auf. Derartige Bilanziersysteme sind beispielsweise in der DE-A-28 38 414 oder der DE-A-197 08 391 beschrieben.

Die in Figur 1 dargestellte Vorrichtung ist im übrigen auch für die Hämofiltration einsetzbar, indem der Durchfluß von Dialysierflüssigkeit durch die Dialysierflüssigkeitskammer 4 unterbunden und gleichzeitig für eine entsprechende Gabe von Substitutionsflüssigkeit an den Patienten gesorgt wird. Im folgenden soll dennoch der Einfachheit halber von "der Dialysevorrichtung" gesprochen werden.

Die Dialysevorrichtung umfaßt ferner eine zentrale Steuer- und Recheneinheit 20, die über Steuerleitungen 21 bis 24 mit der Blutpumpe 6, der Dialysierflüssigkeitspumpe 16, der Ultrafiltrationspumpe 19 und der Bilanziereinrichtung 14 verbunden ist. Die Steuer- und Recheneinheit 20 sendet die Steuerbefehle an die einzelnen Komponenten und empfängt von den Komponenten Daten über die Betriebszustände der Komponenten, beispielsweise die Förderraten der Pumpen, die Bilanzkammertakte etc.

Während der Hämodialysebehandlung wird die Blutkammer 3 von dem Blut des Patienten und die Dialysierflüssigkeitskammer 4 des Dialysators 1 von der Dialysierflüssigkeit durchströmt. Da die Bilanziereinrichtung 14 in den Dialysierflüssigkeitsweg II geschaltet ist, kann nur so viel Dialysierflüssigkeit über die Dialysierflüssigkeitzuführleitung 12 zufließen, wie Dialysierflüssigkeit über die Dialysierflüssigkeitabiuhrleitung 15 abfließen kann. Mit der Ultrafiltrationspumpe 19 kann dem Patienten Flüssigkeit über den Dialysator 1 entzogen werden.

Zur Überprüfung des Dialysierflüssigkeitssystems I auf Undichtigkeiten weist die Dialysevorrichtung eine Einrichtung 25 auf, mit der ein Druckhaltetest durchgeführt werden kann. Hierzu ist die Dialysierflüssigkeitzuführ- und -abführleitung 12, 15 mit einer Bypaßleitung 26 verbunden, in die ein Bypaßventil 27 geschaltet ist. Femer sind stromauf und stromab der Dialysierflüssigkeitskammer 4 des Dialysators 1 in die Dialysierflüssigkeitszuführ- und abführleitung Absperrventile 28, 29 geschaltet. Das Bypaßventil 27 und die Absperrventile 28, 29 sind elektromagnetisch betätigbare Ventile, die über Steuerleitungen 30, 31, 32 mit der Einrichtung zur Überprüfung des Flüssigkeitssystems 25 verbunden sind. Ferner ist ein Drucksensor 33 vorgesehen, der den Druck im zweiten Abschnitt 12b der Dialysierflüssigkeitszufiihrleitung 12 stromauf der Dialysierflüssigkeitskammer 4 mißt. Dieser Drucksensor ist über eine Datenleitung 34 ebenfalls mit der Einrichtung 25 verbunden.

Die Überprüfung des Dialysierflüssigkeitssystems auf Undichtigkeiten erfolgt nach einer Unterbrechung der Dialysebehandlung wie folgt. Die Einrichtung 25 schließt die Absperrventile 28, 29 und öffnet das Bypaßventil 27 für die Dauer des Prüfintervalls T, so daß die Dialysierflüssigkeitskammer 4 vom Dialysierflüssigkeitssystem II abgetrennt ist. Während des Prüfintervalls T überwacht die Einrichtung 25 den Betriebsdruck im Dialysierflüssigkeitssystem II mit dem Drucksensor 33 und vergleicht den Betriebsdruck mit einem vorgegebenen Grenzwert. Der Druck steigt während des Prüfintervalls zunächst an, gleicht sich aus und bleibt im Fall eines intakten Systems stabil. Im Fall eines Lecks dagegen fällt er unter den vorgegebenen Grenzwert ab. Dieser Druckabfall ist ein eindeutiger Indikator dafür, daß das UF-Kontrollsystem nicht mehr integer ist. Wenn der Betriebsdruck also den vorgegebenen Grenzwert unterschreitet, erzeugt die Einrichtung 25 ein Alarmsignal, das eine Alarmeinrichtung 35 über eine Datenleitung 36 empfängt. Die Alarmeinrichtung 35 gibt daraufhin einen akustischen und/oder optischen Alarm. Darüber hinaus kann die gesamte Dialysebehandlung unterbrochen werden. Dieser Druckhaltetest zur Feststellung einer Leckage ist in der DE-A-42 39 937 im einzelnen beschrieben, auf die ausdrücklich Bezug genommen wird.

Neben der Einrichtung 25 zur Überprüfung des Dialysierflüssigkeitssystems mittels eines Druckhaltetests weist die Dialysevorrichtung eine weitere Vorrichtung zur Detektion einer Leckage im Flüssigkeitssystem auf. Diese Vorrichtung weist eine Steuer- und Recheneinheit auf, die Teil der zentralen Steuer- und Recheneinheit 20 der Dialysevorrichtung ist. Es kann aber auch eine separate Steuer- und Recheneinheit vorgesehen sein. Die Steuer- und Recheneinheit weist Mittel 37 zum Erfassen der Druckmesswerte, Mittel 38 zum Bestimmen von Leckraten, Mittel 39 zum Bestimmen eines Leckage-Volumens und Mittel 40 zum Vergleichen des Leckage-Volumens mit einem vorgegebenen Grenzwert auf. Diese Mittel sind in Figur 1 als Teil der zentralen Steuer- und Recheneinheit 20 dargestellt.

Zum Messen des Drucks im Flüssigkeitssystem ist neben dem ersten Drucksensor 33 ein zweiter Drucksensor 41 vorgesehen, der den Druck stromab der Dialysierflüssigkeitskammer 4 im ersten Abschnitt 15a der Dialysierflüssigkeitsabführleitung 15 mißt. Ein dritter Drucksensor 42 mißt den Druck in der Blutabführleitung 7 stromab der Blutkammer 3. Die Drucksensoren 33, 41, 42 sind mit Datenleitungen 43 bis 45 mit der Steuer- und Recheneinheit 20 verbunden.

Das erfindungsgemäße Verfahren zur Detektion von Leckagen wird unter Bezugnahme auf das folgende vereinfachte Modell für den statischen Druck im Flüssigkeitssystem beschrieben.

Es wird angenommen, daß der Dialysierflüssigkeitsfluß $Q_D$ den statischen Druck im Flüssigkeitssystem nicht ändert. Es entstehen nur dynamische Druckabfälle an den Strömungswiderständen, die bei der Betrachtung des statischen Drucks aber nicht berücksichtigt werden. Der statische Druck $P_D$ ist an allen Stellen im Flüssigkeitssystem gleich groß. Der mittlere Druck $P_B$ der Blutpumpe 6 wird als konstant angenommen, wenn der Blutvolumenstrom konstant ist. Da - wie im folgenden ausgeführt wird - die Erfindung auf Druckdifferenzen abstellt, sind die dynamischen Druckabfälle bei konstanten Flüssen ohnehin nicht relevant.

Die Compliance der einzelnen Komponenten des Flüssigkeitssystems, die auf die Verwendung von Schlauchleitungen zurückzuführen ist, wird gedanklich in einer gesamten Compliance C zusammengefaßt, für die gilt:

$$C = \frac{dV_C}{dP_D} = \frac{Q_C}{dP_D / dt} \qquad (1.1)$$

mit

$V_c$ Volumen, das in der Compliance gespeichert wird

$Q_c$ Volumenstrom, der in die Compliance fließt

$P_D$ statischer Druck auf der Dialysatseite

Der Druckabfall an der Membran 2 des Dialysators 1 wird durch folgenden Zusammenhang beschrieben:

$$Q_{TM} = K_{UF} * P_{TM} \qquad (1.2)$$

mit

$$P_{TM} = P_B - P_D \qquad (1.3)$$

wobei

$P_{TM}$  Transmembrandruck [hPa]
$Q_{TM}$  Fluss durch die Membran [ml/h]
$K_{UF}$  Ultrafiltrationskoeffizient

In den Dialysierflüssigkeitskreis II fließt durch die Membran der Volumenstrom $Q_{TM}$ von der Blutseite auf die Dialysatseite. Entzogen werden die Volumenströme durch die Ultrafiltrationspumpe 19 $Q_{UF}$ und gegebenenfalls durch ein Leck $Q_L$. Der resultierende Netto-Volumenstrom fließt in die Compliance $Q_C$ und erzeugt dadurch eine Änderung des statischen Druckes $P_D$. Die Bilanz der einzelnen Volumenströme ergibt:

$$Q_{TM} - Q_{UF} - Q_L = Q_C \qquad (1.4)$$

Mit Gl. (1.1), Gl. (1.2) und Gl. (1.3) folgt:

$$\boxed{C * \frac{dP_{TM}}{dt} + K_{UF} * P_{TM} = Q_{UF} + Q_L} \qquad (1.5)$$

Bei einer konstanten Ultrafiltrationsrate $Q_{UF}$ und einer plötzlich auftretenden Leckage $Q_L$ im Zeitpunkt t=0 ergibt sich die einfache Lösung:

$$P_{TM}(t) = \frac{Q_{UF}}{K_{UF}} + \frac{Q_L}{K_{UF}} * \left(1 - e^{-\frac{t}{\tau}}\right) \qquad (1.6)$$

mit

$$\tau = \frac{C}{K_{UF}} \qquad (1.7)$$

Die Zeitkonstante $\tau$ liegt bei einem harten System (C = 0,01 ml/mmHg) und einem Highflux-Dialysator (z. B. F80 mit $K_{UF}$ = 55 ml/Hg) bei $\tau$ = 0,72 sek. Bei einem Lowflux-Dialysator (z. B. $K_{UF}$ = 2 ml/h / mmHg) und einem weichen System
(C = 0,05 ml/mmHg) ergibt sich für die Zeitkonstante $\tau$ = 90 sek.
Nach Erreichen des neuen Gleichgewichtszustandes $P_{TM}$ (t > 5*$\tau$) hat sich der Druck $P_{TM}$ geändert um:

$$\Delta P_{TM} = P_{TM}(t > 5 * \tau) - P_{TM}(0) = \frac{Q_L}{K_{UF}} \qquad (1.8)$$

6

Eine Leckrate von $Q_L$ =125 ml/h erzeugt bei einem Highflux-Dialysator (z. B. F80 mit $K_{UF}$ = 55 ml/h / mmHg) eine Änderung von $\triangle P_{TM}$ = + 2,5 mmHg und bei einem Lowflux-Dialysator (z. B. $K_{UF}$ = 2 ml/h / mmHg) eine Änderung von $\triangle P_{TM}$ = +62,5 mmHg.

Aus dem obigen Modell lassen sich die folgenden Ergebnisse ableiten.

Die zu erwartenden Druckänderungen sind sehr klein gegenüber den sonstigen Drücken im System. Bei einem plötzlichen Leck ändert sich der Druck über die Dauer von einigen Sekunden bis Minuten, während bei einem schleichenden Leck dieser Verzug vernachlässigt werden kann. Nach Gleichung (1.5) sind die UF-Rate $Q_{UF}$ und die Leckrate $Q_L$ gleichwertige Störungen für den statischen Druck, so daß durch Änderung der UF-Rate die Auswirkung eines Lecks simuliert werden kann.

Aufgrund der Änderung des statischen Druckes $\triangle P$ wird eine Leckrate $Q_L$ errechnet:

$$Q_L(t) = \frac{1}{E} * \Delta P(t) \qquad\qquad (2.1)$$

mit

$$\Delta P(t) = P(t) - P(t - \Delta t) \qquad\qquad (2.2)$$

wobei

P(t)         momentaner Druckwert
P(t - $\Delta$t)     Bezugswert des Druckes
$\Delta$t           Verzögerungszeit des Bezugswertes
E             Empfindlichkeit

Die Empfindlichkeit E hängt von den verwendeten Systemkomponenten ab und wird für jede Kombination aus Dialysatoren, Schlauchsets und Maschinenkomponenten experimentell bestimmt. Diese Werte können in der Steuer- und Recheneinheit 20 hinterlegt sein und vom Benutzer ausgewählt werden.

Es ist auch möglich, am Beginn einer Dialysebehandlung einen vorgegebenen Ultrafiltrationsfluß $Q_{UF}$ zu erzeugen, den Druckabfall $\triangle P$ zu messen und mit $Q_L = Q_{UF}$ die Empfindlichkeit E mit Hilfe von Gleichung (2.1) zu bestimmen.

Die Verzögerungszeit $\triangle t$ ist die zeitliche Differenz der Mittelpunkte der Mittelungsintervalle. Sie sollte größer sein als der Mittelwert der beiden Mittelungszeiten $T_{P(t)}$ und $T_{P(t-\Delta t)}$, damit P(t) und P(t-$\Delta$t) unabhängig voneinander sind:

$$\Delta t \geq (T_{P(t)} + T_{P(t-\Delta t)})/2. \qquad\qquad (2.3)$$

Für die Bildung des Bezugswertes P(t - $\triangle$t) gibt es zwei Möglichkeiten:

1. Der Bezugswert wird dem momentanen Druck P(t) mit der Verzögerungszeit $\Delta$t nachgezogen:

$$\Delta t = const \qquad\qquad (2.4a)$$

2. Der initiale Bezugswert wird beibehalten und nur sporadisch z. B. nach einem Druckhaltetest, auf den momentanen Druckwert gesetzt:

$$\Delta t = t \quad d.\,h. \quad P(t - \Delta t) = P(0) = const \qquad (2.4b)$$

Aufgrund der statistischen Schwankungen der Drucksignale ist das sichere Erkennen eines Leckes erst dann möglich, wenn die Leckrate den Unsicherheitsbereich U überschreitet. Mit Hilfe des Gauß'schen Fortpflanzungsgesetzes von Meßunsicherheiten ergibt sich aus Gl. (2.1) mit Gl. (2.2):

$$U = k_{1-\alpha} * \frac{1}{E} * \sqrt{\sigma_{P(t)}^2 + \sigma_{P(t-\Delta t)}^2} \qquad (2.5)$$

wobei $k_{1-\alpha}$ die Quantile der Normalverteilung für ein Vertrauensniveau von 1-$\alpha$ angibt.

Die Standardabweichung verkleinert sich mit zunehmender Mittelungsdauer T gemäß:

$$\frac{\sigma(T_2)}{\sigma(T_1)} = \sqrt{\frac{T_1}{T_2}} \qquad (2.6)$$

Die Mittelungsdauer des Bezugswertes P(t-$\Delta$t) sollte ca. zehnmal so groß sein wie die des momentanen Druckes P(t). Damit kann dieser Anteil vernachlässigt werden und die Unsicherheit reduziert sich auf:

$$U = k_{1-\alpha} * \frac{\sigma_{P(t)}}{E} \qquad (2.7)$$

Da die Leckraten mit $Q_L < U$ nicht sicher erkannt werden können, muß ein zyklischer Druckhaltetest mit der Periode $T_{zyk.P}$

$$T_{zyk.P} = \frac{500\,ml}{U} \qquad (2.8)$$

durchgeführt werden. Wenn die Periode $T_{zyk.P}$ größer ist als die Behandlungsdauer kann auf den zyklischen Druckhaltetest verzichtet werden. Dies kann in gewissen Grenzen - durch die Verlängerung der Mittelungszeiten $T_{p(t)}$ und $T_{P(t-\Delta t)}$ erreicht werden.

Wenn ein bestimmter Schwellenwert überschritten wird, muß nicht sofort darauf reagiert werden, sondern es kann eine gewisse Zeit abgewartet werden, ob sich der Zustand stabilisiert oder ob es nur ein Artefakt war. Aus Sicht des Patientenschutzes ist das Leckage-Volumen $V_L$ entscheidend, das sich folgendermaßen darstellen läßt:

$$V_L(t) = \int_{t-\Delta t}^{t} Q_L(t')dt' = \frac{1}{E} \int_{t-\Delta t}^{t} \Delta P(t')dt' \qquad (2.9)$$

Nachfolgend wird die Funktionsweise der erfindungsgemäßen Vorrichtung zur Detektion einer Leckage im einzelnen beschrieben.

Vor der Blutbehandlung wird die Integrität des Flüssigkeitssystems mit der Einrichtung 25 nach dem in der DE 42 39 937 A1 beschriebenen Verfahren überprüft. Wenn das System integer ist, wird die Blutbehandlung in Gang gesetzt. Während der Blutbehandlung wird der Druck stromauf ($P_{D1}$) und stromab ($P_{D2}$) mit den Drucksensoren 33 und 41 gemessen. Die Mittel 37 der Steuer- und Recheneinheit berechnen aus dem Druck stromauf und stromab der Dialysierflüssigkeitskammer die Linearkombination $P_{DM} = (P_{D1} + P_{D2})/2$, die von einer Leckage sehr stark beeinflußt wird.

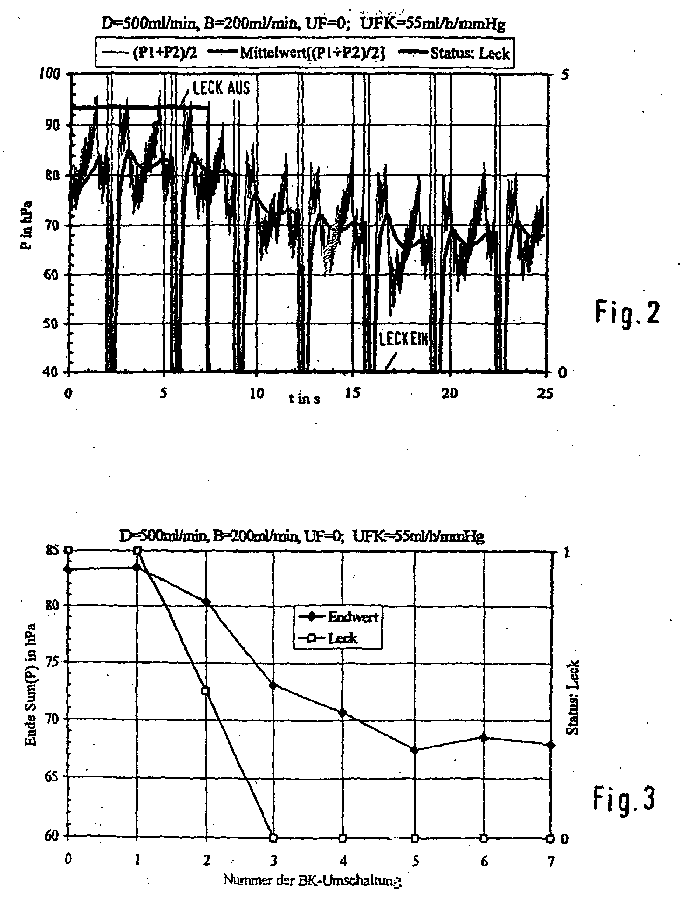

Figur 2 zeigt den zeitlichen Verlauf von $P_{DM}$ und dessen Mittelwert während eines Bilanzkammertaktes vor und nach dem Auftreten eines Lecks, wobei ein Dialysierflüssigkeitsfluß von 500 ml/min, ein Blutfluß von 200 ml/min und eine Ultrafiltrationsrate UF = 0 angenommen wird. Der Ultrafiltrationskoeffizient UFK beträgt 55 ml/h/mmHg.

In Figur 3 ist der Mittelwert von $P_{DM}$ zwischen jeweils zwei Bilanzkammer-Umschaltungen vor und nach dem Auftreten des Lecks bei gleichem Dialysierflussigkeits- bzw. Blutfluß dargestellt. Nach jeder Bilanzkammer-Umschaltung wird wieder mit einer neuen Mittelwertbildung begonnen. Der Mittelwert ist jeweils der Endwert der Mittlung vor der nächsten Bilanzkammer-Umschaltung. Deutlich ist die Abnahme des Drucks nach dem Auftreten der Leckage zu erkennen. Das Mittelungsintervall kann aber auch mehrere Bilanzkammertakte umfassen, um insbesondere bei hohen Dialysierflüssigkeitsflüssen und damit kleinen Mittelungsintervallen eine ausreichende Glättung zu erreichen.

Die Messung des Drucks $P_{DM}$ erfolgt während eines vorgegebenen Zeitraums der Behandlungsdauer, d. h. bis zu dem Zeitpunkt, bis zu dem ein mögliches Leck erkannt wird. Innerhalb des vorgegebenen Zeitraums der Behandlungsdauer werden die Leckraten in vorgegebenen Zeitintervallen, die wiederum mehrere Bilanzkammertakte umfassen können, mit den Mitteln 38 berechnet, in dem der Betrag der Differenz zwischen dem Mittelwert des Drucks $P_{DM}$ in einem nachfolgenden Takt, der mehrere Bilanzkammertakte umfassen kann, und dem Mittelwert des Drucks in einem vorausgehenden Takt bestimmt wird. Während der Blutbehandlung wird dann aus den einzelnen Leckraten in den vorgegebenen Zeitintervallen durch Summenbildung (Integration) das Leckage-Volumen in dem vorgegebenen Zeitraum nach Gleichung (2.9) berechnet. Die Berechnung des Leckage-Volumens erfolgt mit den Mitteln 39 der Steuer- und Recheneinheit 20. Die Mittel 40 der Steuer- und Recheneinheit 20 vergleichen das Leckage-Volumen mit einem vorgegebenen Grenzwert, der beispielsweise 400 ml beträgt.

Für den Fall, daß das Leckage-Volumen über dem Grenzwert liegt, wird auf eine mögliche Undichtigkeit im Flüssigkeitssystem geschlossen. In diesem Fall kann sofort mit der Alarmeinrichtung 35 ein Alarm gegeben werden. Vorzugsweise wird aber die mögliche Undichtigkeit des Systems mit der Einrichtung 25 verifiziert, die den konventionellen Druckhaltetest durchführt. Hierzu unterbricht die Steuer- und Recheneinheit 20 die Blutbehandlung und die Einrichtung 25 trennt den Dialysator vom Dialysierflüssigkeitssystem ab, in dem die Absperrorgane 28, 29 geschlossen und das Bypaßventil 27 geöffnet werden. Erst wenn die Einrichtung 25 eine Undichtigkeit im Flüssigkeitssystem erkennt, gibt die Alarmeinrichtung 35 einen Alarm. Ansonsten wird die Behandlung wieder fortgesetzt, wobei das Flüssigkeitssystem mit der erfindungsgemäßen Vorrichtung wieder kontinuierlich auf mögliche Undichtigkeiten überprüft wird. Sollte die Vorrichtung erneut eine Leckage detektieren, wird die Blutbehandlüng wieder unterbrochen, um die Undichtigkeit wieder mit der Einrichtung 25 zu verifizieren.

Mit dem oben beschriebenen Verfahren bzw. der Vorrichtung können insbesondere plötzlich auftretende Lecks erkannt werden, ohne daß grundsätzlich eine Unterbrechung der Behandlung erforderlich ist. Für die Erkennung von schleichenden Lecks hingegen, hat es sich als vorteilhaft erwiesen, als Bezugswert des Drucks nicht den Druck im vorausgehenden Takt heranzuziehen, sondern den zu Beginn der Behandlung gemessenen Anfangswert des Drucks.

Eine alternative Ausführungsform der erfindungsgemäßen Vorrichtung bzw. des Verfahrens sieht vor, daß nicht der Druck $P_{DM}$, sondern der Transmembrandruck gemessen wird. Mit den zur Verfügung stehenden Drucksensoren wird der Transmembrandruck TMP2 bestimmt, der aus der Differenz des Drucks $P_{B2}$ stromab der Blutkammer 3 des Dialysators 1 und dem Druck $P_{DM}$ berechnet wird, wobei der Druck $P_{B2}$ mit dem Drucksensor 42 gemessen wird. Ansonsten unterscheidet sich diese Ausführungsform nicht von der eingangs beschriebenen Vorrichtung. Alternativ kann auch nur der Druck $P_{B2}$ gemessen werden, wobei die Auswertung der Meßwerte wie bei den vorgenannten Verfahren erfolgt.

Die mit den Sensoren gemessenen Druckwerte sind von Störgrößen überlagert. Figur 4 zeigt den Druck $P_{D1}$ und $P_{D2}$ als Funktion der Zeit, der von den periodischen Störgrößen überlagert ist. Im Zeitsignal der Drücke $P_{D1}$ und $P_{D2}$ dominieren die positiven und negativen Druckspitzen bei der Umschaltung der Bilanzkammereinrichtung 14. Die kleineren Druckschwankungen werden von der Blutpumpe 6 erzeugt. Die Ultrafiltrationspumpe 19 erzeugt bei jedem Hub der Bilanzkammer 13 eine negative Druckspitze.

Die Mittel 37 zum Erfassen der Druckmeßwerte eliminieren die bekannten Störgrößen bevor die Mittelwertbildung erfolgt. Die bekannten Störquellen haben die folgenden Auswirkungen auf die gemessenen Druckwerte.

Die Blutpumpe 6 erzeugt den statischen Druck im System. Der konstante Druckanteil ist von einem Signal überlagert, das auf den Eingriff der Rotoren und den Weitertransportes des Blutes zurückzufuhren ist. Das Maximum des Signals beträgt auf der Blutseite typischerweise 50 hPa und auf der Dialysatseite ca. 10 hPa ($Q_b$ = 300 ml/min). Die Periodendauer liegt in der Größenordnung von 1 sek. Die Druckwerte stromab der Blutkammer 3 des Dialysators 1 werden weniger gestört als die Druckwerte stromauf der Blutkammer. Daher ist die Messung des Drucks PB2 von Vorteil. Da sich mit der Blutvolumenrate auch der mittlere statische Druck im System ändert, wird die Leckage-Überwachung nach einer Änderung der Blutvolumenrate vorzugsweise ausgesetzt bis der neue stationäre Zustand erreicht ist.

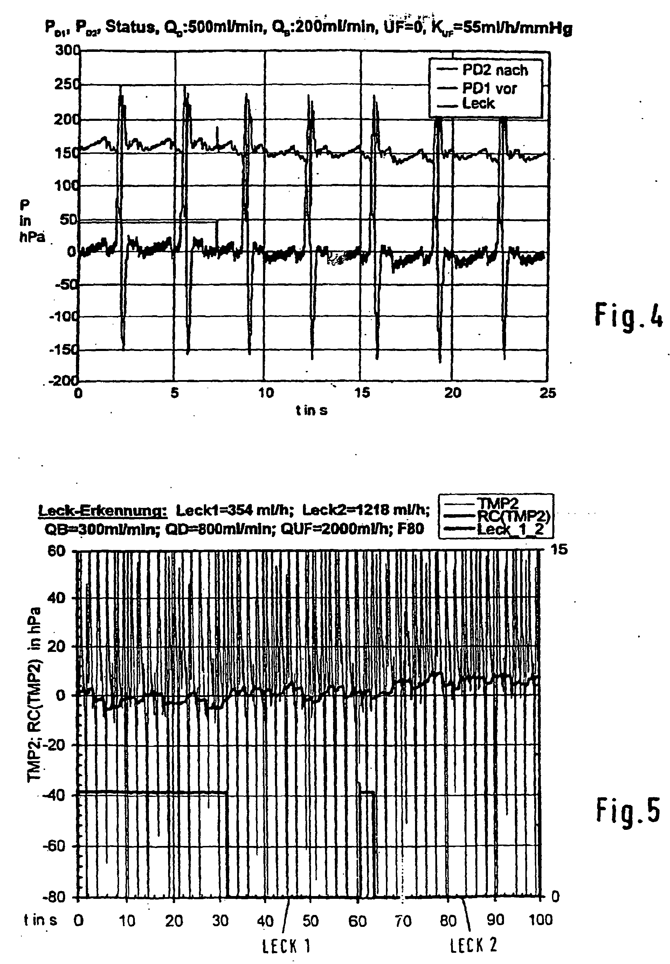

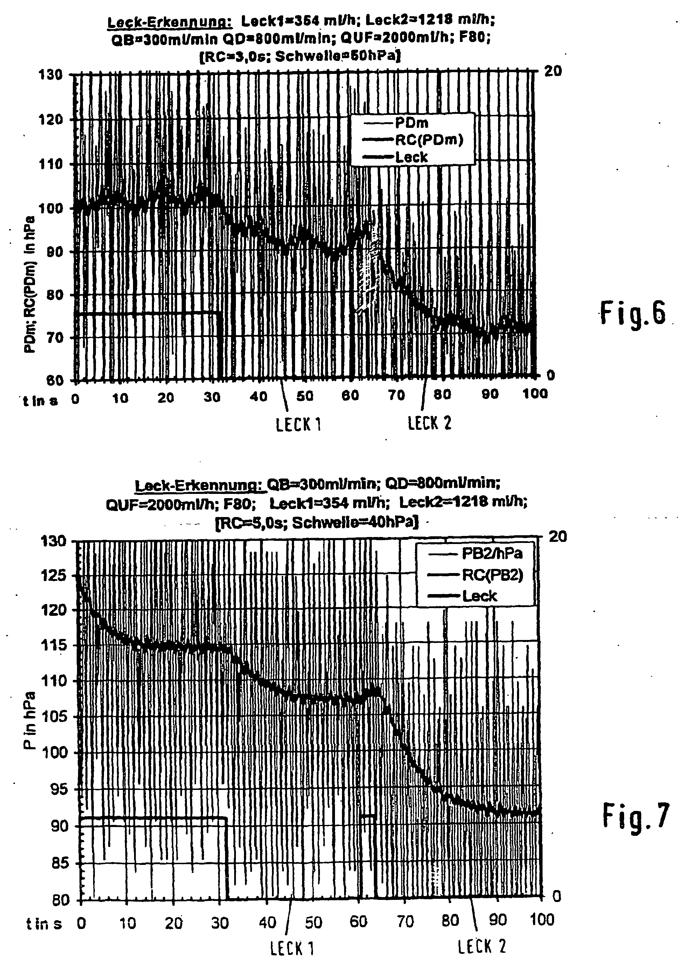

Im folgenden Laborversuch wurden die Signale mit einem digitalen Tiefpassfilter geglättet. In den Figuren 5, 6 und 7 sind jeweils die ungeglätteten Signale TMP2, PDm und PB2 sowie die gefilterten Signale RC(TMP2) und RC(PDm) und RC(PB2) für zwei verschiedene Leckraten dargestellt.

Die Ergebnisse aus den Abbildungen sind in den folgenden Tabellen zusammengefaßt. Die Spalte "STdAbw" enthält die empirische Standardabweichung $\sigma_{P(t)}$ und die Spalte "U" den Unsicherheitsbereich nach Gl. (2.7). In den Tabellen wurde das Vertrauensniveau $1-\alpha$ = 95% und damit $k_{1-\alpha}$ = 2 verwendet.

Die Unsicherheit U stellt die minimale Leckrate dar, die sich von den statistischen Schwankungen der Messgrößen signifikant unterscheidet.

## Tabelle 4.1: Ergebnisse der $P_{TM2}$ - Messung

|        | $Q_L$ [ml/h] | $\Delta P_{TM}$ [hPa] | $1/E_{PTM2}$ [ml/h / hPa] | StdAbw [hPa] | U [ml/h] |
|--------|--------------|----------------------|---------------------------|--------------|----------|
| Leck 1 | 354          | +3,1                 | +114                      | 2,1          | 491      |
| Leck 2 | 1218         | +7,8                 | +156                      | 2,1          | 669      |

## Tabelle 4.2: Ergebnisse der $P_{Dm}$ - Messung

|        | $Q_L$ [ml/h] | $\Delta P_{Dm}$ [hPa] | $1/E_{PDm}$ [ml/h / hPa] | StdAbw [hPa] | U [ml/h] |
|--------|--------------|----------------------|---------------------------|--------------|----------|
| Leck 1 | 354          | -10,7                | -33,1                     | 1,6          | 107      |
| Leck 2 | 1218         | -30,3                | -40,2                     | 1,6          | 129      |

## Tabelle 4.3: Ergebnisse der $P_{B2}$ - Messung

|        | $Q_L$ [ml/h] | $\Delta P_{B2}$ [hPa] | $1/E_{PB2}$ [ml/h / hPa | StdAbw [hPa] | U [ml/h] |
|--------|--------------|----------------------|--------------------------|--------------|----------|
| Leck 1 | 354          | -7,1                 | -49,6                    | 0,48         | 47       |
| Leck 2 | 1218         | -23,0                | -52,9                    | 0,48         | 50       |

In diesem Laborversuch haben die Drucksignale $P_{Dm}$ und $P_{B2}$ die statistische Genauigkeit für eine Überwachung der maximalen Leckrate von 125 ml/h.

**Patentansprüche**

1. Vorrichtung zur Detektion einer Leckage in einem Flüssigkeitssystem einer Blutbehandlungsvorrichtung mit einem extrakorporalen Blutkreislauf (I), wobei die Vorrichtung zur Detektion einer Leckage eine Steuer- und Recheneinheit (20) und Mittel (33,41,42) zum fortlaufenden Messen des Drucks im Flüssigkeitssystem während eines vorgegebenen Zeitraums der Behandlungsdauer aufweist,
   **dadurch gekennzeichnet, daß** die Steuer- und Recheneinheit (20) ferner aufweist:

   Mittel (38) zum Bestimmen der Leckraten in vorgegebenen Zeitintervallen des vorgegebenen Zeitraums der Behandlungsdauer aus der Änderung des Drucks,
   Mittel (39) zum Bestimmen des Leckage-Volumens in dem vorgegebenen Zeitraum der Behandlungsdauer aus den Leckraten und
   Mittel (40) zum Vergleichen des Leckage-Volumens mit einem vorgegebenen Grenzwert.

2. Vorrichtung nach Anspruch 1, **dadurch gekennzeichnet, daß** Mittel (38) zum Bestimmen der Leckraten derart ausgebildet sind, daß zur Bestimmung der Änderung des Drucks während des vorgegebenen Zeitraums in aufeinander folgenden Takten fortlaufend jeweils ein Druckwert bestimmt und mit einem Bezugswert des Drucks verglichen wird.

**3.** Vorrichtung nach Anspruch 2, **dadurch gekennzeichnet, daß** der Bezugswert des Drucks der jeweils in einem vorhergehenden Takt bestimmte Druckwert ist.

**4.** Vorrichtung nach Anspruch 2, **dadurch gekennzeichnet, daß** der Bezugswert des Drucks konstant ist.

**5.** Vorrichtung nach einem der Ansprüche 2 bis 4, **dadurch gekennzeichnet, daß** der Druckwert ein Mittelwert der in einem Mittelungsintervall gemessenen Druckwerte ist.

**6.** Vorrichtung nach einem der Ansprüche 1 bis 5, **dadurch gekennzeichnet, daß** eine Alarmvorrichtung (35) vorgesehen ist, die einen Alarm gibt, wenn das Leckage-Volumen den vorgegebenen Grenzwert überschreitet.

**7.** Vorrichtung nach einem der Ansprüche 1 bis 6, **dadurch gekennzeichnet, daß** eine Einrichtung (25) zur Überprüfung des Flüssigkeitssystems auf Undichtigkeiten mittels eines Druckhaltetests in dem Flüssigkeitssystem vorgesehen ist, wobei die Steuer- und Recheneinheit (20) derart ausgebildet ist, daß die Blutbehandlung unterbrochen und zur Überprüfung des Flüssigkeitssystems auf Undichtigkeiten die Einrichtung zur Durchführung eines Druckhaltetests aktivierbar ist, wenn das Leckage-Volumen den vorgegebenen Grenzwert überschreitet.

**8.** Vorrichtung nach Anspruch 7, **dadurch gekennzeichnet, daß** die Alarmeinrichtung (35) derart ausgebildet ist, daß bei der Feststellung von Undichtigkeiten mittels des Druckhaltetests ein Alarm gegeben wird.

**9.** Vorrichtung nach einem der Ansprüche 1 bis 8, **dadurch gekennzeichnet, daß** die Blutbehandlungsvorrichtung einen von einer semipermeablen Membran (2) in eine Blutkammer (3) und eine Dialysierflüssigkeitskammer (4) unterteilten Dialysator (1) mit einer Blutzuführleitung (5) zum Zuführen von Blut in die und einer Blutabführleitung (7) zum Abführen von Blut aus der Blutkammer und einer Dialysierflüssigkeitszuführleitung (12) zum Zuführen von Dialysierflüssigkeit in die und einer Dialysierflüssigkeitsabführleitung (15) zum Abführen von Dialyisierflüssigkeit aus der Dialysierflüssigkeitskammer aufweist.

**10.** Vorrichtung nach Anspruch 9, **dadurch gekennzeichnet, daß** die Mittel (37) zum fortlaufenden Messen des Drucks im Flüssigkeitssystem einen den Druck in der Dialysierflüssigkeitszuführleitung (12) stromauf und/oder in der Dialysierflüssigkeitsabführleitung (15) stromab des Dialysators (1) und/oder einen den Druck in der Blutabführleitung (7) stromab des Dialysators messenden Drucksensor (33, 41, 42) aufweisen.

**Claims**

**1.** A device for the detection of a leakage in a fluid system of a blood treatment apparatus with an extracorporeal blood circuit (1), whereby the device for the detection of a leakage has a control and computation unit (20) and means (33, 41, 42) for the continuous measurement of the pressure in the fluid system during a predetermined space of time of the treatment period,
**characterised in that** the control and computation unit (20) also has:

means (38) for the determination of the leakage rate at predetermined time intervals of the predetermined space of time in the treatment period from the change in the pressure,
means (39) for the determination of the leakage volume in the predetermined space of time of the treatment period from the leakage rates and
means (40) for comparing the leakage volume with a preset limiting value.

**2.** The device according to claim 1, **characterised in that** means (38) for the determination of the leakage rate are designed in such a way that, in order to determine the change in the pressure during the predetermined space of time, a pressure value is determined continuously in successive cycles and compared with a reference value of the pressure.

**3.** The device according to claim 2, **characterised in that** the reference value of the pressure is the pressure value determined each time in a preceding cycle.

**4.** The device according to claim 2, **characterised in that** the reference value of the pressure is constant.

**5.** The device according to any one of claims 2 to 4, <u>**characterised in that**</u> the pressure value is an average value of the pressure values measured in an averaging interval.

**6.** The device according to any one of claims 1 to 5, <u>**characterised in that**</u> an alarm device (35) is provided, which emits an alarm when the leakage volume exceeds the preset limiting value.

**7.** The device according to any one of claims 1 to 6, <u>**characterised in that**</u> a device (25) is provided for checking the fluid system for leakages by means of a pressure hold test, whereby the control and computation unit (20) is designed in such a way that the blood treatment is interrupted and the device for performing the pressure hold test can be activated to check the fluid system for leakages when the leakage volume exceeds the preset limiting value.

**8.** The device according to claim 7, <u>**characterised in that**</u> the alarm device (35) is designed in such a way that an alarm is emitted when leakages are ascertained by means of the pressure hold test.

**9.** The device according to any one of claims 1 to 8, <u>**characterised in that**</u> the blood treatment apparatus has a dialyser (1) divided by a semipermeable membrane (2) into a blood chamber (3) and a dialysing fluid chamber (4), with a blood supply line (5) for the supply of blood into the blood chamber and a blood discharge line (7) for the discharge of blood from the blood chamber and a dialysing fluid supply line (12) for the supply of dialysing fluid into the dialysing fluid chamber and a dialysing fluid discharge line (15) for the discharge of dialysing fluid from the dialysing fluid chamber.

**10.** The device according to claim 9, <u>**characterised in that**</u> the means (37) for the continuous measurement of the pressure in the fluid system have a pressure sensor (33, 41, 42) that measures the pressure in the dialysing fluid supply line (12) upstream of the dialyser (1) and/or in the dialysing fluid discharge line (15) downstream of said dialyser and/or one that measures the pressure in the blood discharge line (7) downstream of the dialyser.

**Revendications**

**1.** Dispositif destiné à détecter une fuite dans un circuit de liquide d'un appareil de traitement du sang doté d'une circulation sanguine extracorporelle (1), le dispositif destiné à détecter une fuite comprenant une unité de commande et de calcul (20) et des moyens (33, 41, 42) destinés à mesurer en continu la pression dans le circuit de liquide pendant un laps de temps prédéterminé de la durée du traitement,
**caractérisé en ce que** l'unité de commande et de calcul (20) comprend en outre :

des moyens (38) pour déterminer les taux de fuite dans des intervalles de temps prédéterminés du laps de temps prédéterminé de la durée du traitement à partir de la modification de la pression,
des moyens (39) pour déterminer le volume de fuite dans le laps de temps prédéterminé de la durée du traitement à partir des taux de fuite et
des moyens (40) destinés à comparer le volume de fuite à une valeur seuil prédéterminée.

**2.** Dispositif selon la revendication 1, **caractérisé en ce que** les moyens (38) pour déterminer les taux de fuite sont conçus de telle sorte que pour déterminer la modification de la pression pendant le laps de temps prédéterminé en continu au cours de cycles successifs, respectivement une valeur de pression est déterminée et est comparée à une valeur de référence de la pression.

**3.** Dispositif selon la revendication 2, **caractérisé en ce que** la valeur de référence de la pression est la valeur de pression respectivement déterminée au cours d'un cycle antérieur.

**4.** Dispositif selon la revendication 2, **caractérisé en ce que** la valeur de référence de la pression est constante.

**5.** Dispositif selon l'une quelconque des revendications 2 à 4, **caractérisé en ce que** la valeur de pression est une valeur moyenne des valeurs de pression mesurées dans un intervalle de calcul de moyenne.

**6.** Dispositif selon l'une quelconque des revendications 1 à 5, **caractérisé en ce qu'**il est prévu un dispositif avertisseur (35), qui déclenche une alarme lorsque le volume de fuite dépasse la valeur seuil prédéterminée.

**7.** Dispositif selon l'une quelconque des revendications 1 à 6, **caractérisé en ce qu'**il est prévu un système (25) pour contrôler les pertes d'étanchéité du circuit de liquide au moyen d'un test de maintien de pression dans le circuit de liquide, l'unité de commande et de calcul (20) étant conçue de telle sorte que le traitement du sang est interrompu et, pour contrôler les pertes d'étanchéité du circuit de liquide, le système pour réaliser un test de maintien de pression peut être activé lorsque le volume de fuite dépasse la valeur seuil prédéterminée.

8. Dispositif selon la revendication 7, **caractérisé en ce que** le système avertisseur (35) est conçu de telle sorte que, lorsque des pertes d'étanchéité sont constatées au moyen d'un test de maintien de pression, l'alarme est déclenchée.

9. Dispositif selon l'une quelconque des revendications 1 à 8, **caractérisé en ce que** le dispositif de traitement du sang comprend un dialyseur (1) divisé par une membrane semi-perméable (2) en un compartiment réservé au sang (3) et un compartiment réservé au dialysat (4), doté d'une conduite d'amenée du sang (5) destinée à amener le sang dans le compartiment réservé au sang et d'une conduite d'évacuation du sang (7) destinée à évacuer le sang du compartiment réservé au sang et d'une conduite d'amenée du dialysat (12) destinée à amener le dialysat dans le compartiment réservé au dialysat et d'une conduite d'évacuation du dialysat (15) destinée à évacuer le dialysat du compartiment réservé au dialysat.

10. Dispositif selon la revendication 9, **caractérisé en ce que** les moyens (37) pour mesurer en continu la pression dans le circuit de liquide comprennent un capteur de pression (33, 41, 42) mesurant la pression dans la conduite d'amenée du dialysat (12) en amont et/ou dans la conduite d'évacuation du dialysat (15) en aval du dialyseur (1) et/ou mesurant la pression dans la conduite d'évacuation du sang (7) en aval du dialyseur.

Fig.1

D=500ml/min, B=200ml/min, UF=0; UFK=55ml/h/mmHg

—— (P1+P2)/2    —— Mittelwert[(P1+P2)/2]    —— Status: Leck

Fig.2

D=500ml/min, B=200ml/min, UF=0; UFK=55ml/h/mmHg

—◆— Endwert
—□— Leck

Fig.3

Nummer der BK-Umschaltung

P_D1, P_D2, Status, Q_D:500ml/min, Q_B:200ml/min, ÜF=0, K_UF=55ml/h/mmHg

**Fig. 4**

Leck-Erkennung: Leck1=354 ml/h; Leck2=1218 ml/h; QB=300ml/min; QD=800ml/min; QUF=2000ml/h; F80

**Fig. 5**

16

**Leck-Erkennung:** Leck1=354 ml/h; Leck2=1218 ml/h;
QB=300ml/min QD=800ml/min; QUF=2000ml/h; F80;
[RC=3,0s; Schwelle=50hPa]

Fig.6

**Leck-Erkennung:** QB=300ml/min; QD=800ml/min;
QUF=2000ml/h; F80; Leck1=354 ml/h; Leck2=1218 ml/h;
[RC=5,0s; Schwelle=40hPa]

Fig.7